# EUROPEAN PATENT APPLICATION

(11) **EP 1 938 766 A1**
(43) Date of publication of application: **02.07.2008**
(21) Application number: 07254884.5
(22) Date of filing: 17.12.2007
(51) Int. Cl.: A61B 17/88, B25B 23/10, A61B 17/86

(54) **Cannulated bone screw and cannulated driver for the implantation thereof**

(30) Priority: 15.12.2006 US 611198
(71) Applicant: Zimmer Technology, Inc., Warsaw, IN 46580 (US)
(72) Inventor: Fritzinger, Daniel D., Warsaw Indiana 46582 (US)
(74) Representative: Mays, Julie

(57) **Abstract**

A cannulated driver including an elongate body having an internal wall defining a longitudinal bore extending substantially the entire length of the elongate body. A cannulated bone screw including a threaded shaft, a head, and a longitudinal bore extending entirely through the threaded shaft and the head of the cannulated bone screw. In one exemplary embodiment, the exterior surface of the head of the cannulated bone screw may include an indentation formed therein. The indentation may be any shape, such as a semi-circular shape. The indentation may be configured to mate with a corresponding projection on the internal wall of a cannulated driver to rotationally lock the cannulated bone screw relative to the cannulated driver.

## Description

### BACKGROUND

### 1. Field of the Invention.

The present invention relates to orthopedic devices, and, more particularly, to cannulated bone screws and to cannulated drivers for the implantation of the same.

### 2. Description of the Related Art.

Orthopedic bone plates may be used to maintain different parts of fractured bones substantially stationary relative to one another. A bone plate may be formed as an elongate body having apertures extending therethrough and may be positioned to extend across the fracture line in a bone. Once positioned, bone screws may be inserted through the apertures to secure the bone plate to the fragments of the bone. To facilitate the proper alignment of the bone screws with the apertures of a bone plate and to ease the insertion thereof, a guide wire may be used. A guide wire is a flexible wire which is inserted into a patient's bone at the point where the surgeon intends for the cannulated bone screw to be positioned. After inserting the guide wire, a cannulated bone screw, i.e., a screw having a bore formed therein which extends entirety through the screw along its longitudinal axis, may be received on the guide wire. With the cannulated bone screw received on the guide wire, the cannulated bone screw may be moved along the guide wire to contact the bone at the intended position.

Once positioned, the cannulated bone screw may be implanted into the bone. To implant the bone screw, a driver may be used. The driver may also be cannulated to allow the driver to be received on and moved along the guide wire in the same manner as the cannulated bone screw. To engage and rotationally lock the cannulated bone screw, the driver may have a head configured to engage a corresponding recess formed within the head of the cannulated bone screw. For example, the head of the driver may have a hexagonal external surface and the cannulated bone screw may have a corresponding internal hexagonal recess.

### SUMMARY

The present invention relates to orthopedic devices, and, more particularly, to cannulated bone screws and to cannulated drivers for the implantation of the same. The cannulated driver of the present invention includes an elongate body having an internal wall defining a longitudinal bore extending substantially the entire length of the elongate body. Additionally, the cannulated bone screw includes a threaded shaft, a head, and a longitudinal bore extending entirely through the threaded shaft and the head of the cannulated bone screw. In one exemplary embodiment, the exterior surface of the head of the cannulated bone screw may include a flat portion. The flat portion may be configured to mate with a correspondingly flat on the internal wall of the cannulated driver to rotationally lock the cannulated bone screw relative to the cannulated driver.

In one exemplary embodiment, the head of the cannulated driver further includes an O-ring retained therein. In another exemplary embodiment, the head of the cannulated driver further includes a plurality of resiliently deformable fingers. The resiliently deformable fingers are configured to extend over the exterior surface of the screw head and resiliently retain the cannulated bone screw within the longitudinal bore of the cannulated driver. In another exemplary embodiment, the exterior surface of the screw head includes an indentation formed therein. The indentation may be any shape, such as a semi-circular or "V" shape. In this embodiment, the cannulated driver includes a projection extending from the internal wall defining the longitudinal bore. The projection is configured to matingly engage the indentation in the screw head to rotationally lock the cannulated bone screw relative to the cannulated driver.

Advantageously, by utilizing the cannulated bone screws and corresponding cannulated drivers of the present invention, the cross section of the cannulated driver can be substantially larger. As a result, the cannulated driver can withstand substantially greater forces imparted thereto during implantation of a cannulated bone screw. In contrast, the cross-section of previous cannulated drivers, which engage the interior of the head of the cannulated bone screw with the exterior of the body of the cannulated driver, are restricted by the cross sectional area of the recess in the head of the cannulated bone screw. For example, a previous cannulated driver configured to mate with an internal hexagonal recess in the head of a cannulated bone screw can have a cross section no greater than the cross section of the hexagonal recess.

Additionally, when a screw having a threaded head is utilized with the cannulated drivers of the present invention, the cannulated drivers will begin to disengage from the cannulated bone screw once the cannulated driver contacts a bone plate. For example, when the cannulated driver begins to seat the cannulated bone screw within an aperture in a bone plate, the cannulated driver will be prevented from advancing with the screw due to the interaction with the surface of the bone plate. As a result, the screw will begin to disengage from the cannulated driver until the cannulated bone screw is completely disengaged and is no longer rotationally locked with respect to the cannulated driver. A surgeon can then remove the cannulated driver and the guide wire to additionally seat the cannulated bone screw using a solid driver.

In one form thereof, the present invention provides a cannulated driver for driving a cannulated bone screw having a shaft and head into a bone, the cannulated bone screw receivable on a guide wire attached to the bone for guiding the cannulated bone screw into the bone, the cannulated driver further receivable on the guide wire and engageable for rotation with the cannulated bone screw to drive the cannulated bone screw into the bone, the cannulated driver including: an elongate body having an interior wall defining a longitudinal bore, the longitudinal bore extending through the elongate body, the longitudinal bore sized to receive the guide wire therethrough, the longitudinal bore further sized to receive the head of the cannulated bone screw, the internal wall of the elongate body configured to matingly engage the head of the cannulated bone screw, whereby the cannulated bone screw is rotationally locked with respect to the elongate body when the cannulated bone screw is matingly engaged with the interior wall of the elongate body.

In another form thereof, the present invention provides an orthopedic system including: a cannulated driver having an elongate body and an internal wall defining a longitudinal bore, the longitudinal bore extending through the elongate body; and a cannulated bone screw having a head, a shaft, and a longitudinal bore extending through the head and the shaft of the cannulated bone screw, the head configured for mating engagement with the internal wall of the cannulated driver, whereby mating engagement of the head of the cannulated bone screw and the internal wall of the cannulated driver rotationally locks the cannulated bone screw with respect to the cannulated driver.

In yet another form thereof, the present invention provides a method of inserting a cannulated bone screw into a bone, including the steps of: inserting a guide wire into a bone; receiving a cannulated bone screw having a head and a corresponding cannulated driver having a longitudinal bore on the guide wire; positioning the head of the cannulated bone screw within the longitudinal bore of the cannulated driver to rotationally lock the cannulated bone screw with respect to the cannulated driver; and screwing the cannulated bone screw into the bone with the cannulated driver.

### BRIEF DESCRIPTION OF THE DRAWINGS

The above-mentioned and other features and advantages of this invention, and the manner of attaining them, will become more apparent and the invention itself will be better understood by reference to the following description of an embodiment of the invention taken in conjunction with the accompanying drawings, wherein:

Fig. 1 is a perspective view of a cannulated bone screw according to one exemplary embodiment of the present invention;

Fig. 2 is an exploded view of the cannulated bone screw of Fig. 1, a cannulated driver according to one exemplary embodiment of the present invention, and a guide wire;

Fig. 3 is a fragmentary, partial cross sectional view of the cannulated driver and cannulated bone screw of Fig. 2 taken along line 3-3 of Fig. 2;

Fig. 4 is a perspective view of a cannulated driver according to another exemplary embodiment with the cannulated bone screw of Fig. 1;

Fig. 5 is a fragmentary, partial cross sectional view of the assembly of Fig. 4 taken along ling 5-5 of Fig. 4;

Fig. 6 is a perspective view of a cannulated bone screw according to another exemplary embodiment;

Fig. 7 is an exploded perspective view of the cannulated bone screw of Fig. 6 and a cannulated driver according to another exemplary embodiment;

Fig. 8 is a cross sectional view of the cannulated driver of Fig. 7 taken along line 8-8 of Fig. 7;

Fig. 9 is a cannulated bone screw according to yet another exemplary embodiment;

Fig. 10 is an exploded perspective of the cannulated bone screw of Fig. 8 with a cannulated driver according to yet another exemplary embodiment; and

Fig. 11 is a front view of a solid driver configured to mate with the bone screws of the present invention.

Corresponding reference characters indicate corresponding parts throughout the several views. The exemplifications set out herein illustrate preferred embodiments of the invention and such exemplifications are not to be construed as limiting the scope of the invention in any manner.

### DETAILED DESCRIPTION

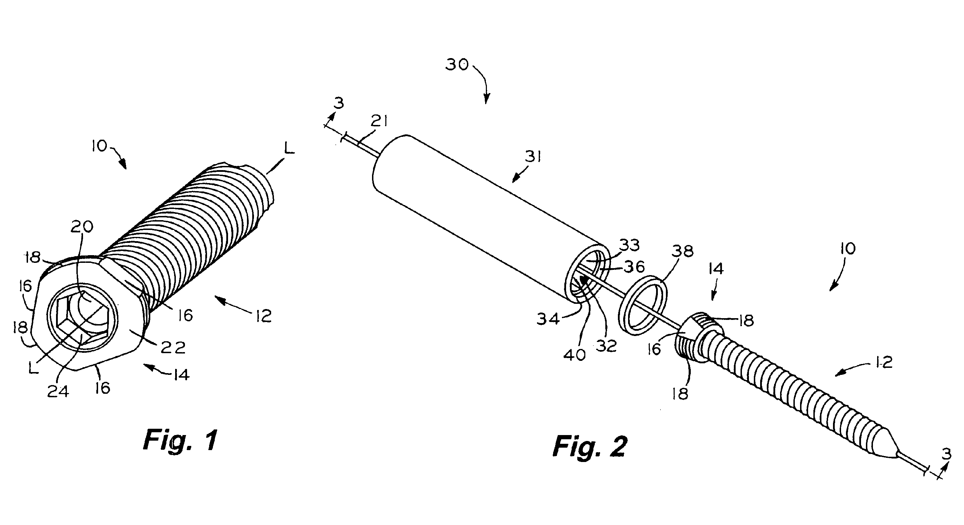

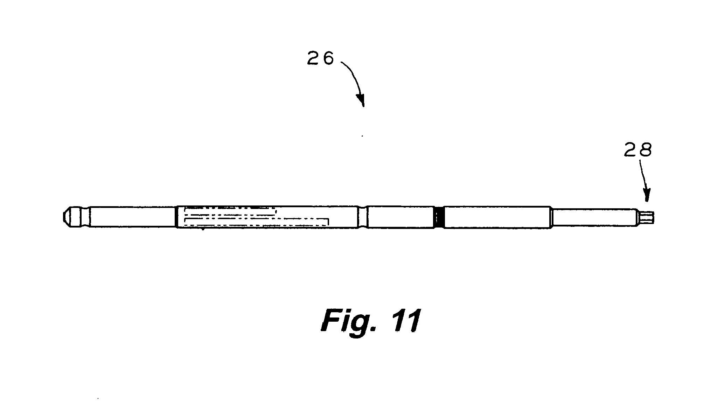

Fig. 1 depicts cannulated bone screw 10 according to an exemplary embodiment of the present invention. Cannulated bone screw 10 includes threaded shaft 12 and head 14. Head 14 includes flat portions 16 and threaded portions 18. Longitudinal bore 20 extends entirely through head 14 and threaded shaft 12 of cannulated bone screw 10 along longitudinal axis L of cannulated bone screw 10. Longitudinal bore 20 forms the cannulation of cannulated bone screw 10, which allows objects, such as guide wire 21 (Fig. 2), to pass therethrough. A portion of longitudinal bore 20 adjacent top surface 22 of head 14 forms hexagonal counter bore 24. Hexagonal counter bore 24 is configured to mate with an orthopedic tool, such as solid driver 26, shown in Fig. 11, having hexagonal head 28 at one end thereof. Hexagonal head 28 is configured to engage the walls defining hexagonal counter bore 24 to rotationally lock solid driver 26 to cannulated bone screw 10.

Referring to Fig. 2, cannulated driver 30 is depicted according to one exemplary embodiment of the present invention. Cannulated driver 30 includes elongate body 31 having an internal wall 33 which defines longitudinal bore 32. Longitudinal bore 32 extends through substantially the entire length of cannulated driver 30. In the same manner as longitudinal bore 20 of cannulated bone screw 10, longitudinal bore 32 of cannulated driver 30 allows objects, such as guide wire 21, to pass therethrough. Adjacent to end surface 34 of cannulated driver 30 is groove 36 formed in internal wall 33. Groove 36 is sized to receive and retain O-ring 38 therein. O-ring 38 provides compressive engagement with cannulated bone screw 10 to retain head 14 of cannulated bone screw 10 within longitudinal bore 32, as described in detail below. Additionally, internal wall 33 includes flats 40 extending from end surface 34. Flats 40 are configured to matingly engage flat portions 16 of head 14 to rotationally lock cannulated bone screw 10 relative to cannulated driver 30.

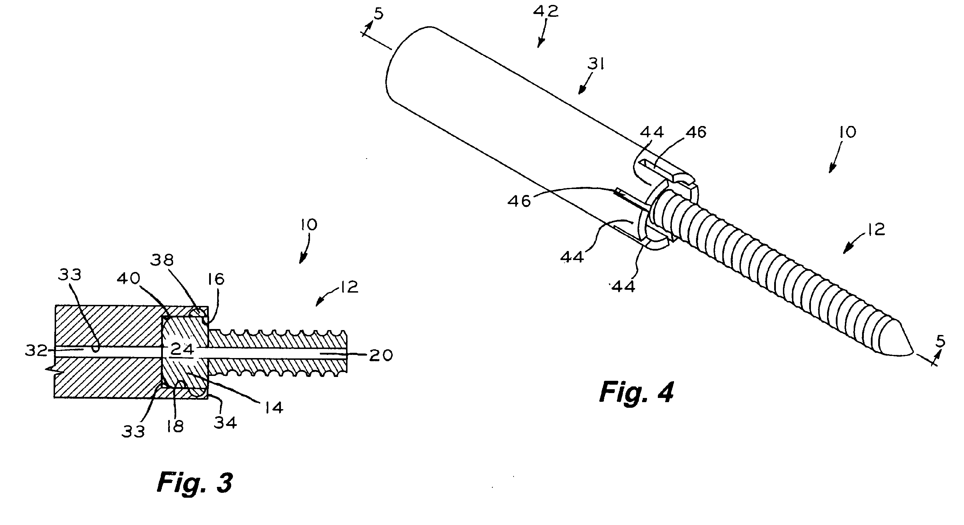

Referring to Fig. 3, cannulated bone screw 10 is shown matingly engaged with cannulated driver 30 and rotationally locked with respect thereto, as described above. Additionally, O-ring 38 is resiliently deformed by the presence of head 14 of cannulated bone screw 10 within longitudinal bore 32 of cannulated driver 30. As a result, O-ring 38 exerts a force against head 14 of cannulated bone screw 10 sufficient to retain cannulated bone screw 10 within longitudinal bore 32 of cannulated driver 30. Specifically, when head 14 of cannulated bone screw 10 is tapered, rounded, or oval and is inserted into cannulated driver 30, O-ring 38 expands to allow the major diameter of head 14 to pass therethrough. Once the major diameter of head 14 has passed through O-ring 38, O-ring 38 relaxes to engage a diameter of head 14 that is smaller than the major diameter of head 14, retaining cannulated bone screw 10 within cannulated driver 30. Therefore, movement of cannulated driver 30 results in corresponding movement of cannulated bone screw 10. Advantageously, this allows a surgeon to seat cannulated driver 30 on head 14 of cannulated bone screw 10 and remove cannulated bone screw 10 from a screw caddy or other storage device simply by moving cannulated driver 30. This frees the surgeon from having to manually retain cannulated bone screw 10 within longitudinal bore 32 of cannulated driver 30 during movement thereof and lessens the likelihood of dropping or otherwise misplacing cannulated bone screw 10.

Referring to Fig. 2, during an operation, a surgeon may position one end of guide wire 21 through an aperture in a bone plate positioned adjacent a bone. The end of guide wire 21 may then be advanced into a bone to secure guide wire 21 thereto. Once guide wire 21 is secured in a bone, the surgeon may engage head 14 of cannulated bone screw 10 with internal wall 33 defining longitudinal bore 32 of cannulated driver 30, as described in detail above. Then, cannulated bone screw 10 and cannulated driver 30 may then be received on guide wire 21 by positioning guide wire 21 within longitudinal bore 20 and longitudinal bore 32 of cannulated bone screw 10 and cannulated driver 30, respectively. The surgeon may then advance cannulated bone screw 10 and cannulated driver 30 along guide wire 21 until cannulated bone screw 10 contacts the bone in which guide wire 21 is secured. Once in this position, the surgeon may rotate cannulated driver 30 to begin threading shaft 12 of cannulated bone screw 10 into the bone along guide wire 21 positioned within the aperture of a bone plate, as described above.

As cannulated driver 30 is rotated, cannulated bone screw 10 will continue to advance into the bone. As the surgeon continues to rotate cannulated driver 30, either manually or with the use of additional tools, end surface 34 of cannulated driver 30 may contact the outer surface of the bone plate. At this point, continued rotation of cannulated driver 30 will continue to result in corresponding rotation of cannulated bone screw 10. However, as cannulated bone screw 10 is advance further into the bone, head 14 of cannulated bone screw 10 will begin to disengage from flats 40 and O-ring 38 of cannulated driver 30. Specifically, the exterior surface of the bone plate will prevent additional translation of cannulated driver 30 along guide wire 21 toward the bone. However, the continued rotation of cannulated driver 30 will continue to advance cannulated bone screw 10 into the bone, ultimately disengaging cannulated driver 30 and cannulated bone screw 10.

Once cannulated bone screw 10 has disengaged from cannulated driver 30, the surgeon can remove cannulated driver 30 and use solid driver 26 (Fig. 11) to additionally seat cannulated bone screw 10 within the aperture of the bone plate, as necessary. Specifically, referring to Fig. 11, hexagonal head 28 of solid driver 26 is inserted within hexagonal counter bore 24 of cannulated bone screw 10 to rotationally lock cannulated bone screw 10 with respect to solid driver 26. As a result, rotation of solid driver 26 results in corresponding rotation of cannulated bone screw 10 and allows a surgeon, either manually or by utilizing additional tools, to seat cannulated bone screw 10 within the aperture of the bone plate.

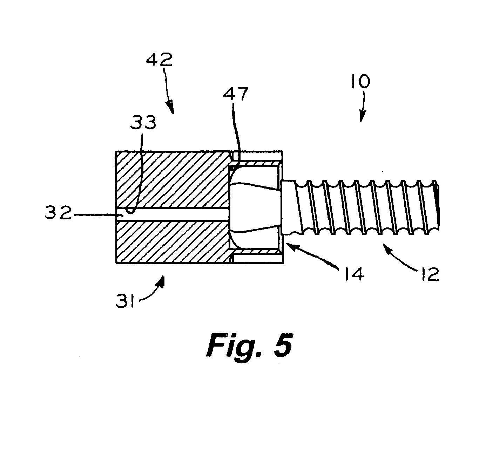

Figs. 4 and 5 depict cannulated driver 42 according to another exemplary embodiment. As described and depicted herein, cannulated driver 42 includes several features which are substantially similar or identical to corresponding features of cannulated driver 30 and identical reference numerals are utilized to identify substantially similar or identical components therebetween. As shown in Figs. 4 and 5, cannulated driver 42 includes resiliently deformable fingers 44 separated by slots 46. Referring to Fig. 5, resiliently deformable fingers 44 engage the exterior surface of head 14 of cannulated bone screw 10. As shown in Fig. 5, several of resiliently deformable fingers 44 include interior flat surface 47, which matingly engages with flat portions 16 of head 14. As described above with specific reference to cannulated driver 30, resiliently deformable fingers 44 engage flat portions 16 to rotationally lock cannulated bone screw 10 and cannulated driver 42 together. To seat head 14 of cannulated bone screw 10 within through longitudinal bore 32 of cannulated driver 42, cannulated driver 42 is forced downward over head 14 and toward shaft 12 of cannulated bone screw 10. As fingers 44 move over head 14 they deform outwardly. This outward deformation results in fingers 44 exerting a force against head 14 of cannulated bone screw 10 to retain the same therein.

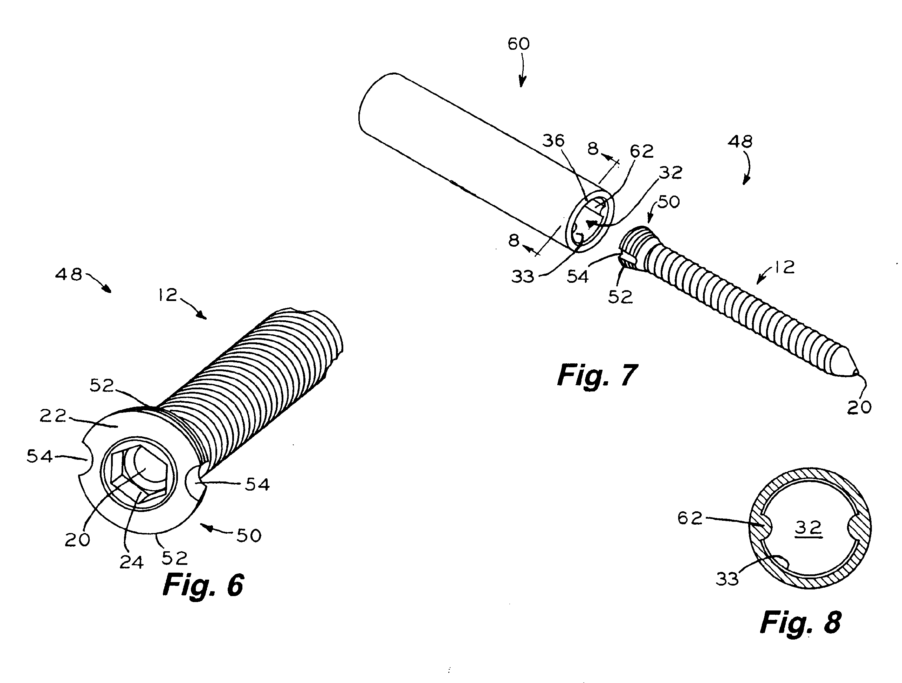

Referring to Figs. 6 and 7, cannulated bone screw 48 is depicted according to another exemplary embodiment. Cannulated bone screw 48 has several features which are identical or substantially identical to corresponding features of cannulated bone screw 10 of Figs. 1-5, discussed in detail herein, and identical reference numerals have been used to identify identical or substantially identical features therebetween. Cannulated bone screw 48 includes threaded shaft 12, head 50, and longitudinal bore 20. Head 50 includes threaded portions 52 and indentations 54 formed therein. While indentations 54 are depicted herein as semi-circular, indentations 54 may be any shape, such as "V" shape. Longitudinal bore 20 includes hexagonal counter bore 24 positioned adjacent top surface 22 of head 50.

Referring to Figs. 7 and 8, cannulated driver 60 is depicted according to another exemplary embodiment. Cannulated driver 60 has several features which are identical or substantially identical to corresponding features of cannulated driver 30 of Figs. 2 and 3, discussed in detail herein, and identical reference numerals have been used to identify identical or substantially identical features therebetween. Cannulated driver 60 interior wall 33 defining longitudinal bore 32. Projections 62 are formed on interior wall 33 and extend from longitudinal bore 32 radially inwardly. Projections 62 are configured to matingly engage indentations 54 of head 50 of cannulated bone screw 48. Specifically, projections 62, when engaged with indentations 54, rotationally lock cannulated bone screw 48 with respect to cannulated driver 60. In another exemplary embodiment, O-ring 38 (Fig. 2) may be positioned within a groove (not shown) formed within interior wall 33 to facilitate the retention of cannulated bone screw 48 within longitudinal bore 32.

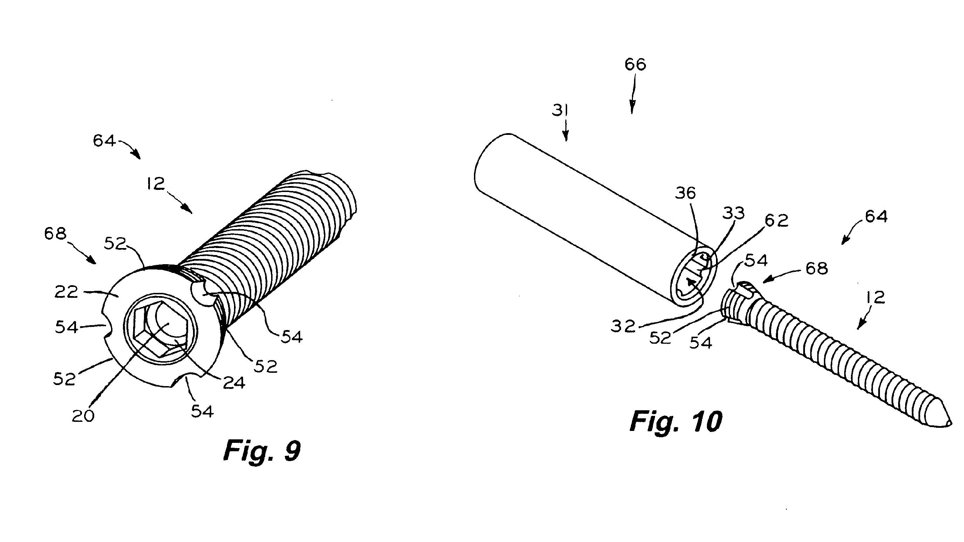

Figs. 9 and 10 depict cannulated bone screw 64 and cannulated driver 66 according to another exemplary embodiment. Cannulated bone screw 64 and cannulated driver 66 are substantially identical to cannulated bone screw 48 and cannulated driver 60 of Figs. 6 and 7, respectively and identical reference numerals have been used to identify identical or substantially identical features therebetween. In contrast to cannulated bone screw 48 of Figs. 6 and 7, cannulated bone screw 64 includes an additional indentation 54. Specifically, indentations 54 of cannulated bone screw 64 are separated by 120 degrees, while indentations 54 of cannulated bone screw 48 are separated by 180 degrees. Similarly, cannulated driver 66 includes an additional projection 62 configured to matingly engage indentations 54 of cannulated bone screw 64. As described in detail above with reference to cannulated driver 60, projections 62 of cannulated driver 66 are configured to matingly engage indentations 54 on head 68 of cannulated bone screw 64. Once engaged, cannulated bone screw 64 is rotationally locked to cannulated driver 66 and cannulated driver 66 may be utilized to seat cannulated bone screw 64 within a bone, as described in detail above. While specific embodiments of the invention have been described herein as having specific numbers of indentations 54 and corresponding projections 62 arranged in specific geometric orientations, any number of indentations 54 and projections 62 may be utilized in any geometrical configuration.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A cannulated driver for driving a cannulated bone screw having a shaft and head into a bone, the cannulated bone screw receivable on a guide wire attached to the bone for guiding the cannulated bone screw into the bone, the cannulated driver further receivable on the guide wire and engageable for rotation with the cannulated bone screw to drive the cannulated bone screw into the bone, the cannulated driver comprising:
an elongate body having an interior wall defining a longitudinal bore, said longitudinal bore extending through said elongate body, said longitudinal bore sized to receive the guide wire therethrough, said longitudinal bore further sized to receive the head of the cannulated bone screw, said internal wall of said elongate body configured to matingly engage the head of the cannulated bone screw, whereby the cannulated bone screw is rotationally locked with respect to said elongate body when the cannulated bone screw is matingly engaged with said interior wall of said elongate body.

2. The cannulated driver of Claim 1, further comprising an O-ring and said internal wall further comprising a groove formed therein, said O-ring configured to be received within said groove, whereby said O-ring facilitates retention of the cannulated bone screw within said longitudinal bore of said cannulated driver when the head of the cannulated bone screw is received therein.

3. The cannulated driver of Claim 1, wherein said internal wall further comprises a projection extending therefrom, said projection configured to matingly engage a corresponding indentation in the head of the cannulated bone screw.

4. The cannulated driver of Claim 3, wherein said projection comprises a semi-circular shape.

5. The cannulated driver of Claim 3, further comprising an O-ring and said internal wall further comprising a groove formed therein, said O-ring configured to be received within said groove, whereby said O-ring facilitates retention of the cannulated bone screw within said longitudinal bore of said cannulated driver when the head of the cannulated bone screw is received therein.

6. The cannulated driver of Claim 1, wherein said internal wall further comprises a flat, said flat configured to matingly engage a corresponding flat portion on the head of the cannulated bone screw.

7. The cannulated driver of Claim 1, wherein said elongate body further comprises a resiliently deformable finger, whereby said resiliently deformable finger is deformed to receive the head of the cannulated bone screw within said longitudinal bore.

8. An orthopedic system comprising:
a cannulated driver having an elongate body and an internal wall defining a longitudinal bore, said longitudinal bore extending through said elongate body; and
a cannulated bone screw having a head, a shaft, and a longitudinal bore extending through said head and said shaft of said cannulated bone screw, said head configured for mating engagement with said internal wall of said cannulated driver, whereby mating engagement of said head of said cannulated bone screw and said internal wall of said cannulated driver rotationally locks said cannulated bone screw with respect to said cannulated driver.

9. The orthopedic system of Claim 8, further comprising an O-ring and said internal wall of said cannulated driver further comprising a groove formed therein, said O-ring configured to be received within said groove, whereby said O-ring facilitates retention of said cannulated bone screw within said longitudinal bore of said cannulated driver when said head of said cannulated bone screw is received within said longitudinal bore of said cannulated driver.

10. The orthopedic system of Claim 8, wherein said cannulated driver further comprises a projection extending from said internal wall and said cannulated bone screw further comprises a corresponding indentation formed in said head, said projection of said cannulated driver configured to matingly engage said corresponding indentation of said cannulated bone screw when said head of said cannulated bone screw is received within said longitudinal bore of said cannulated driver.

11. The orthopedic system of Claim 10, wherein said projection comprises a semi-circular shape.

12. The orthopedic system of Claim 8, wherein said internal wall of said cannulated driver further comprises a flat and said head of said cannulated bone screw further comprises a corresponding flat portion formed thereon, said flat of said cannulated driver configured to matingly engage said corresponding flat portion on said head of said cannulated bone screw when said head is received within said longitudinal bore of said cannulated driver.

13. The orthopedic system of Claim 8, wherein said cannulated driver further comprises a resiliently deformable finger, whereby said resiliently deformable finger is deformable to receive said head of said cannulated bone screw within said longitudinal bore of said cannulated driver.

14. The method of Claim 16, further comprising the step of seating the head of the cannulated bone screw within the aperture of the bone plate with the solid driver.
